Europäisches Patentamt

European Patent Office

Office européen des brevets

(19)

(11) Veröffentlichungsnummer: **0 275 470 B1**

(12) **EUROPÄISCHE PATENTSCHRIFT**

(45) Veröffentlichungstag der Patentschrift: **06.03.91**

(51) Int. Cl.⁵: **C07C 233/45**

(21) Anmeldenummer: **87118393.5**

(22) Anmeldetag: **11.12.87**

(54) **N-substituierte, Estergruppen enthaltende Acrylamide.**

(30) Priorität: **22.12.86 DE 3644009**

(43) Veröffentlichungstag der Anmeldung:
**27.07.88 Patentblatt 88/30**

(45) Bekanntmachung des Hinweises auf die
Patenterteilung:
**06.03.91 Patentblatt 91/10**

(84) Benannte Vertragsstaaten:
**AT BE CH DE FR GB IT LI LU NL SE**

(56) Entgegenhaltungen:
**EP-A- 0 020 000**
**US-A- 4 159 383**

**CHEMICAL ABSTRACTS, Band 104, Nr. 14, 7.
April 1986, Seite 28, Zusammenfassung Nr.
110583r, Columbus, Ohio, US; C.L. McCOR-
MICK et al.: "Water-soluble copolymers. 14.
Potentiometric and turbidimetric studies of
water-soluble copolymers of acrylamide:
comparison of carboxylated and sulfonated
copolymers"**

(73) Patentinhaber: **Chemie Linz Gesellschaft
m.b.H.**
**St.Peter-Strasse 25**
**A-4021 Linz(AT)BE**

(72) Erfinder: **Schwarz, Franz-Thomas, Dipl.-Ing.
Dr.**
**Tavernstrasse 15**
**A-4493 Wolfern(AT)**
Erfinder: **Humer, Gerhild**
**Lilienthalstrasse 3/6**
**A-4020 Linz(AT)**

(74) Vertreter: **Kunz, Ekkehard, Dr.**
**Chemie Holding AG Patentwesen St. Peter-
Strasse 25**
**A-4021 Linz(AT)**

## Beschreibung

Die Erfindung betrifft neue N-substituierte, Estergruppen enthaltende Acrylamide, ein Verfahren zu ihrer Herstellung, sowie ihre Verwendung.

N-substituierte Acrylamide bzw. deren Polymerisate werden beispielsweise zur Herstellung von Flockungsmitteln für Abwässer, von Textilhilfsmitteln oder von hydrophilen, quellfähigen Polymeren verwendet. Die Herstellung eines solchen N-substituierten Acrylamids, das eine freie Carbonsäuregruppe enthält, wird beispielsweise im Journal of Polymer Science, Vol 10, 3311 - 3315 (1972) beschrieben. Dabei wird 3-Acrylamido-3-methylbutancarbonsäure durch Umsetzung von Dimethylacrylsäure mit Acrylnitril in Gegenwart von Schwefelsäure mit einer Ausbeute von nur 32 % erhalten.

Es wurde nun überraschenderweise gefunden, daß bei der Umsetzung von Derivaten des Acrylnitrils mit substituierten Alkencarbonsäureestern neue N-substituierte Acrylamide, die Carbonsäureestergruppen enthalten, mit sehr guten Ausbeuten erhalten werden. Durch anschließende Hydrolyse können diese Ester praktisch quantitativ in die freie Carbonsäure oder ein beliebiges Salz dieser Säure umgewandelt werden, welche in bekannter Weise zu den gewünschten, Carboxylgruppen enthaltenden Polyacrylamiden polymerisiert werden können. Es ist aber auch möglich, die neuen Verbindungen direkt, unter Erhaltung der Estergruppen zu entsprechenden Estergruppen enthaltenden Polyacrylamiden zu polymerisieren.

Gegenstand der Erfindung sind demnach N-substituierte, Estergruppen enthaltende Acrylamide der allgemeinen Formel I des Formelblattes, in der $R_1$ Wasserstoff oder eine Methylgruppe, $R_2$, $R_3$ und $R_4$ unabhängig voneinander einen Alkylrest mit 1 - 4 C-Atomen und n eine der Zahlen von 1 - 4 bedeuten. Ein bevorzugtes Acrylamid, bei dem in der allgemeinen Formel I des Formelblattes $R_1$ Wasserstoff, $R_2$, $R_3$ und $R_4$ eine Methylgruppe und n = 1 bedeuten, ist der 3-Acrylamido-3-methylbutancarbonsäuremethylester. Ein weiteres bevorzugtes Acrylamid, bei dem sämtliche Substituenten in der allgemeinen Formel I des Formelblattes Methylgruppen darstellen und n = 1 ist, ist der 3-Methacrylamido-3-methylbutancarboansäuremethylester.

Die erfindungsgemäßen Acrylamide werden durch Umsetzung von Acrylnitril oder Methacrylnitril mit substituierten Alkencarbonsäureestern der allgemeinen Formel II des Formelblattes hergestellt. Beispielsweise erhält man den 3-Acrylamido-3-methylbutancarbonsäuremethylester durch Reaktion von Acrylnitril mit Dimethylacrylsäuremethylester. Die Umsetzung von Acrylnitril oder Methacrylnitril mit den entsprechenden Alkencarbonsäureestern wird unter den Bedingungen der Ritter-Reaktion in stark saurem Medium unter Verwendung von konzentrierten Säuren, wie z. B. Schwefelsäure, Phosphorsäure, Salpetersäure, Methansulfonsäure, Trifluormethansulfonsäure ausgeführt. Bevorzugt ist die Verwendung von Schwefelsäure. Die Säurekonzentration beträgt etwa 70 bis 98 %, wobei bei Schwefelsäure eine Konzentration von 96 % besonders bevorzugt ist. Pro Mol Alkencarbonsäureester werden bevorzugt 1 bis 3 Mol Säure und 0,2 bis 2 Mol, besonders bevorzugt 0,4 bis 1,0 Mol Wasser angewandt. Die im Verlauf der exothermen Reaktion entstehende Wärme wird vorteilhaft durch Kühlung abgeführt, um bei höheren Temperaturen eventuell vorkommende Nebenreaktionen zu verhindern. Bevorzugt wird eine Reaktionstemperatur von Raumtemperatur bis 50 °C eingehalten. Besonders bevorzugt wird die Reaktion bei Temperaturen von 40 bis 45 °C durchgeführt.

Zur weiteren Aufarbeitung wird das saure Reaktionsgemisch unter Kühlung mit Wasser verdünnt, bevorzugt gießt man das Reaktionsgemisch auf Eiswasser, wobei die Temperatur vorteilhaft nicht über 40 °C, besonders bevorzugt nicht über 20 °C ansteigen sollte. Anschließend wird das Gemisch neutralisiert, beispielsweise durch Zugabe von Ammoniakwasser oder Natronlauge. Die Isolierung des N-substituierten Acrylamids erfolgt daraus beispielsweise durch Extraktion mit einem Lösungsmittel wie z. B. Toluol oder Butylacetat und anschließendes Abdampfen des Lösungsmittels. Im Falle von höhermolekularen in Wasser schwer löslichen Produkten, werden diese einfach abfiltriert, gewaschen und getrocknet.

Die Hydrolyse der neuen, N-substituierten, Estergruppen enthaltenden Acrylamide wird in üblicher Weise durchgeführt und ist praktisch quantitativ. Sie erfolgt beispielsweise durch Erwärmen der Ester in wäßrigem alkalischem oder saurem Medium auf 40 bis 80 °C, bevorzugt auf 50 bis 60 °C, wobei für die alkalische Hydrolyse bevorzugt Natronlauge, für die saure Hydrolyse bevorzugt Schwefelsäure, Methansulfonsäure, Phosphorsäure oder Salpetersäure eingesetzt werden. Die durch saure Hydrolyse bzw. durch Ansäuern des Salzes nach der alkalischen Hydrolyse erhaltene freie Säure der allgemeinen Formel III des Formelblattes wird aus der wäßrigen Phase beispielsweise mit einem organischen Lösungsmittel, wie z. B. Butylacetat oder Toluol, extrahiert und anschließend das Extraktionsmittel abgetrennt. Die Salze der N-substituierten Acrylamide können durch Neutralisation der freien Säure mit einem Alkali- oder Erdalkalihydroxid bzw. mit Ammoniak in reiner Form isoliert werden.

Die Charakterisierung der neuen Verbindungen erfolgte mit Hilfe der $^1$H-NMR-Spektren in DMSO.

**Beispiel 1:**

In einem 250 ml Reaktionskolben wurden 0,5 mol (57,07 g) Dimethylacrylsäuremethylester, 0,5 mol (26,5 g) Acrylnitril und 4 g $H_2O$ vorgelegt. Anschließend wurden 1,2 mol (122,6 g) konzentrierte Schwefelsäure (96 %ig) zudosiert. Während der exothermen Reaktion ließ man die Temperatur auf 45°C ansteigen. Nach beendeter Säuredosierung wurde noch 90 min bei 45°C gerührt. Das dickflüssige Reaktionsgemisch wurde anschließend in 200 ml Eiswasser gegossen, wobei man die Temperatur nicht über 20°C ansteigen ließ. Die stark saure Lösung wurde durch Zugabe von Ammoniak-Wasser auf pH 6 neutralisiert, wobei die Temperatur durch Kühlung bei 10 - 20°C gehalten wurde. Zuletzt wurde die Lösung mit Toluol extrahiert, und das Extraktionsmittel am Rotavapor abdestilliert, wobei 71,3 g (77 % d. Th.) 3-Acrylamido-3-methylbutancarbonsäuremethylester als weiße Substanz mit einem Schmelzpunkt von 45 - 48°C erhalten wurden.
$^1$H-NMR (ppm) : 7,70 (s), 6,20 - 5,39 (m), 3,45 (s), 2,73 (s), 1,24 (s).

**Beispiel 2:**

Es wurde wie im Beispiel 1 verfahren, anstelle von Acrylnitril wurden jedoch 0,5 mol (33,5 g) Methacrylnitril eingesetzt. Nach Aufarbeiten des Reaktionsgemisches erhielt man 42 g (84,3 % d. Th.) 3-Methacrylamido-3-methylbutancarbonsäureester als hellgelbes viskoses Öl.
$^1$H-NMR (ppm) : 6,45 (s), 5,66 und 5,29 (d), 2,74 (s), 1,94 (s), 1,48 (s), 1,47 (s).

**Beispiel 3:**

Es wurde wie in Beispiel 1 verfahren, anstelle von Ammoniakwasser für die Neutralisation wurde jedoch 25 %ige Natronlauge verwendet. Nach der Neutralisation wurde mit Natronlauge auf pH 10 eingestellt und auf 60°C erwärmt. Durch kontinuierliches Zugeben weiterer Natronlauge wurde der pH-Wert auf 10 gehalten. Nach ca. 3 Stunden war die Hydrolyse des Esters beendet. Durch Zugabe von Schwefelsäure wurde der pH-Wert auf 2,5 eingestellt. Die so gebildete freie 3-Acrylamido-3-methylbutancarbonsäure wurde drei Mal mit je 50 ml Butylacetat aus der wäßrigen Phase extrahiert. Nach Abdestillieren des Extraktionsmittels und Umkristallisation aus Butylacatat verblieben 32 g (68, 2 % d. Th.) 3-Acrylamido-3-methylbutancarbonsäure als weißes kristallines Produkt mit einem Schmelzpunkt von 93°C.
$^1$H-NMR (ppm) : 11,13 (s), 7,81 (s), 6,31 - 5,50 (m), 2,74 (s), 1,36 (s).

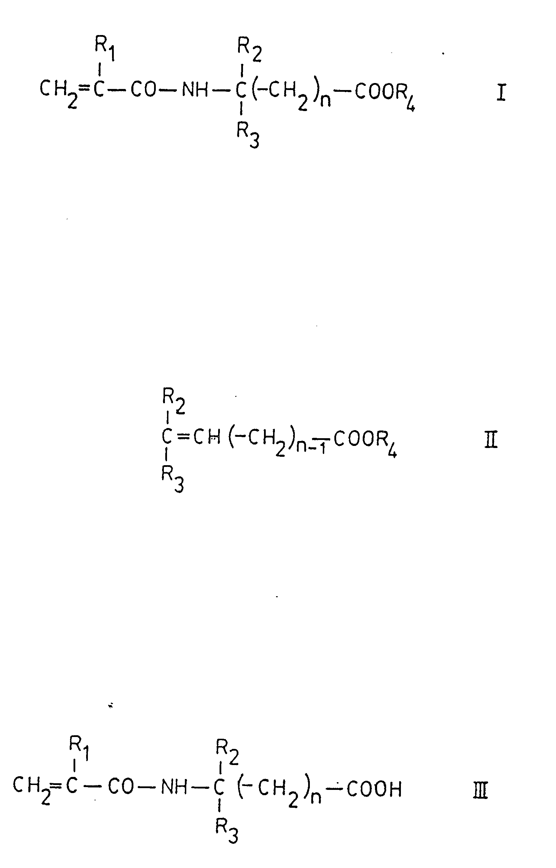

**Ansprüche**

1. N-substituierte, Estergruppen enthaltende Acrylamide der allgemeinen Formel I des Formelblattes, in der $R_1$ Wasserstoff oder eine Methylgruppe, $R_2$, $R_3$ und $R_4$ unabhängig voneinander einen Alkylrest mit 1 - 4 C-Atomen und n eine der Zahlen von 1 - 4 bedeuten.

2. 3-Acrylamido-3-methylbutancarbonsäuremethylester.

3. 3-Methacrylamido-3-methylbutancarbonsäuremethylester.

4. Verfahren zur Herstellung N-substituierter, Estergruppen enthaltender Acrylamide gemäß einem der Ansprüche 1 - 3, dadurch gekennzeichnet, daß man Acrylnitril oder Methacrylnitril, mit einer Verbindung der allgemeinen Formel II des Formelblattes, in der die Substituenten die oben angegebene Bedeutung haben, in Gegenwart von Säuren umsetzt, wobei der erhaltene Ester der allgemeinen Formel I des Formelblattes anschließend gegebenenfalls durch Hydrolyse in die freie Säure oder eines ihrer Salze übergeführt werden kann.

5. Verwendung von N-substituierten, Esterguppen enthaltenden Acrylamiden gemäß einem der Ansprüche 1 - 3 der allgemeinen Formel I des Formelblattes zur Herstellung der freien Säure der allgemeinen Formel III des Formelblattes, in der die Substituenten die oben angegebene Bedeutung haben, bzw. deren Salzen durch saure oder alkalische Hydrolyse.

**Claims**

1. N-substituted acrylamides, containing ester groups, of the general formula I in the formula sheet in which $R_1$ denotes hydrogen or a methyl group, $R_2$, $R_3$ and $R_4$ independently of one another denote an alkyl radical having 1 - 4 C atoms and n denotes one of the numbers 1 - 4.

2. Methyl 3-acrylamido-3-methylbutane carborylate.

3. Methyl 3-methacrylamido-3-methylbutane carboxylate.

4. Process for the preparation of N-substituted acrylamides, containing ester groups, according to one of Claims 1 to 3, characterized in that acrylonitrile or methacrylonitrile is reacted in the presence of acids with a compound of the general formula II in the formula sheet in which the substituents have the meaning indicated above, it being possible subsequently to convert the resulting ester of the general formula I in the formula sheet, if appropriate by hydrolysis, into the free acid or one of its salts.

5. Use of N-substituted acrylamides, containing ester groups, according to one of Claims 1 to 3, of the general formula I in the formula sheet for the preparation of the free acid of the general formula III in the formula sheet in which the substituents have the meaning indicated above, or salts thereof, by acid or alkaline hydrolysis.

**Revendications**

1. Acrylamides N-substitués renfermant des groupes ester, selon la formule générale I qui figure dans la planche unique de dessins annexée, dans laquelle $R_1$ représente un atome d'hydrogène ou un groupe méthyle, $R_2$, $R_3$, et $R_4$, indépendants les uns des autres, représentent un reste alkyle de 1 à 4 atomes de carbone et n est un nombre compris entre 1 et 4.

2. Ester méthylique de l'acide 3-acrylamido-3-méthylbutane carboxylique.

3. Ester méthylique de l'acide 3-méthacrylamido-3-méthylbutane carboxylique.

4. Procédé de préparation d'acrylamides N-substitués renfermant des groupes esters selon l'une des revendications 1 à 3, caractérisé en ce qu'on fait réagir en présence d'acide l'acrylnitrile ou le méthacrylnitrile avec le composé de formule générale II qui figure dans la planche unique de dessins annexée, dans laquelle les substituants ont la signification indiquée ci-dessus, l'ester obtenu de formule générale I qui figure dans la planche unique de dessins annexée, est transformé à la fin le cas échéant par hydrolyse en acide libre ou en un de ses sels.

5. Utilisation des acrylamides N-substitués renfermant des groupes ester selon une des revendications 1 à 3, de la formule générale I qui figure dans la planche unique de dessins annexée, pour la préparation d'acides libres de formule générale III qui figure dans la planche unique de dessins annexée, dans laquelle les substituants ont la signification indiquée ci-dessus, ou bien de leurs sels par hydrolyse acide ou alcaline.

$$\underset{R_3}{\overset{R_1}{CH_2{=}C{-}CO{-}NH{-}\underset{|}{\overset{|}{C}}(-CH_2)_n{-}COOR_4}} \qquad I$$

$$\underset{R_3}{\overset{R_2}{\overset{|}{C}{=}CH\,(-CH_2)_{n-1}COOR_4}} \qquad II$$

$$\underset{R_3}{\overset{R_1}{CH_2{=}C{-}CO{-}NH{-}\underset{|}{\overset{|}{C}}(-CH_2)_n{-}COOH}} \qquad III$$